(19) **Europäisches Patentamt**
**European Patent Office**
**Office européen des brevets**

(11) **EP 4 394 083 A1**

(12) **EUROPEAN PATENT APPLICATION**
published in accordance with Art. 153(4) EPC

(43) Date of publication:
**03.07.2024 Bulletin 2024/27**

(21) Application number: **23841734.9**

(22) Date of filing: **21.02.2023**

(51) International Patent Classification (IPC):
**C23C 28/04** $^{(2006.01)}$

(52) Cooperative Patent Classification (CPC):
**Y02E 60/10**

(86) International application number:
**PCT/CN2023/077426**

(87) International publication number:
**WO 2024/016656 (25.01.2024 Gazette 2024/04)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **22.07.2022 CN 202210868209**

(71) Applicants:
• **Shenzhen Asia Kidney Rebuilding Medical
Technology Ltd.
Shenzhen, Guangdong 518000 (CN)**
• **Guangzhou Asia Kidney Rebuilding Medical
Technology Ltd.
Guangzhou, Guangdong 510700 (CN)**

(72) Inventors:
• **CHEN, Zhonghui
Shenzhen, Guangdong 518000 (CN)**
• **ZHAO, Xi
Shenzhen, Guangdong 518000 (CN)**
• **ZHUANG, Guangzhen
Shenzhen, Guangdong 518000 (CN)**
• **LUO, Ruiguang
Shenzhen, Guangdong 518000 (CN)**
• **ZHENG, Lixin
Shenzhen, Guangdong 518000 (CN)**
• **WANG, Heng
Shenzhen, Guangdong 518000 (CN)**
• **QIU, Jiang
Shenzhen, Guangdong 518000 (CN)**

(74) Representative: **Kehl, Ascherl, Liebhoff & Ettmayr
Patentanwälte Partnerschaft mbB
Emil-Riedel-Straße 18
80538 München (DE)**

(54) **ALUMINA FILM, AND PREPARATION METHOD THEREFOR AND USE THEREOF**

(57) Disclosed is an alumina membrane, its preparation method and application. A method for preparing an alumina membrane comprises the following steps: carrying out constant voltage anodizing treatment on a surface on one side of an aluminum sheet to obtain an alumina membrane with a porous structure on the surface on one side; removing pure aluminum on an other side of the alumina membrane by a physical processing method, and carrying out pore-enlarging treatment to obtain a membrane with interconnected pores on both sides; and depositing a silicon coating on the surface of the membrane on the side that has been physically processed to obtain the alumina membrane. According to the disclosure, the pure aluminum on the other side is etched by physical processing method after the aluminum on one side is oxidized, so as to avoid an absorbability of an alumina crystal form formed by chemical reagent corrosion on a platelet membrane protein. Specifically, depositing silicon coating on alumina membrane effectively reduces the adsorption of proteins on membrane surface, and improves the filtration efficiency and clearance ability of membrane toward protein factors.

FIG. 2

EP 4 394 083 A1

**Description**

**TECHNICAL FIELD**

The present disclosure relates to the technical field of an alumina membrane, and in particular to an alumina membrane, a preparation method and use thereof.

**BACKGROUND**

Sepsis is a systemic inflammatory syndrome caused by the invasion of bacteria or other pathogenic microorganisms into the body. In addition to systemic inflammatory syndrome and primary infection lesions, organ hypoperfusion also exists in critically ill patients. With the occurrence of sepsis, the interaction of endogenous inflammatory mediators, including vasoactive substances, cytokines, chemokines, oxygen free radicals, acute phase response substances, bioactive lipids, products of plasma enzyme system, and blood fibrin is in a state of imbalance. Due to the uncontrolled regulation of the body micro-environmental, an extensive damage outbreaks in organs and the whole system.

Nephritis and renal failure caused by sepsis are commonly treated with hemofiltration in clinical practice. U.S. Patent (Publication No. US6653131B2) discloses a method for treating systemic inflammatory response syndrome (SIRS) by contacting the patient's body fluids with renal tubular cells in vitro. The renal tubular cells are seeded on porous microbeads (made of microporous gelatin and collagen-coated dextran) to prepare a renal tubule assist device. The blood is filtered by a hemofilter, and then infused back to the patient through the tubule-assist device. This treatment relies on the active uptake of inflammatory factors from the patient's blood by the renal tubules in the assist device. The uptake and transport of inflammatory factors by cells belongs to the life activities of cells, and its uptake and transport efficiency is related to the cellular microenvironment. Existing techniques have been used to attach renal cells to the dialysis membrane to achieve a resorption function. However, when cells are cultured on conventional artificial semi-permeable membranes (e.g., PES, PS, PP), the growth of renal cells is slow, the cell viability will decrease under long-term culture, and subsequently weakens the ability of protein uptake and transport.

**SUMMARY**

The present disclosure aims at improving the viability of renal tubular cells on an alumina membrane, and subsequently promoting the protein transport capacity of renal tubular cells. According to the present disclosure, a preparation process of an alumina membrane is improved, and the prepared alumina membrane provides a suitable microenvironment for the growth of renal tubular cells, which can relatively improve the viability of renal tubular cells and promote their uptake and transport of proteins, as well as the clearance of inflammatory factors. The alumina membrane attached to the renal tubular cells according to the present disclosure can be used for hemofiltration to treat sepsis, and it is not necessary to use additional biological materials (such as porous microbeads) to prepare renal tubular assist devices.

A first object of the present disclosure is to provide an alumina membrane, a second object is to provide a method for preparing such alumina membrane, a third object is to provide an alumina biofilm, and a fourth object is to provide such alumina membrane and an application of alumina biofilm.

In order to achieve the above objects, the technical solution used in the present disclosure is as follows:
A first aspect of the present disclosure provides a preparation method for alumina membrane, comprising the following steps:

> (1) carrying out constant voltage anodizing treatment on a surface on one side of an aluminum sheet to obtain an alumina membrane with a porous structure on the surface on one side;
> (2) removing pure aluminum on an other side of the alumina membrane prepared in step (1) by a physical processing method, and carrying out pore-enlarging treatment to obtain a membrane with interconnected pores on both sides; and
> (3) depositing a silicon coating on the surface of the membrane on the side that has been physically processed in step (2) to obtain the alumina membrane.

Preferably, in the preparation method of such alumina membrane, in step (1), a pretreatment is carried out on the aluminum sheet prior to constant voltage anodizing treatment, which includes the following steps: degreasing and electrochemical polishing of the aluminum sheet. Further preferred, the degreasing is specifically as follows: the aluminum sheet is immersed in ethanol for 3-7 minutes. The electrochemical polishing is as follows: putting the aluminum sheet into an electrolytic cell, and carrying out electrochemical polishing by a constant current method. The electrolyte is an ethanol solution of 100 g/L of phosphoric acid and 40 g/L of polyethylene glycol-400. The polishing treatment is conducted at a current value of 2.63 A, and a voltage does not exceed 130 V for about 15 minutes. After polishing, the aluminum sheet with a bright and flat surface is obtained.

Preferably, in the preparation method of the alumina membrane, in step (1), a voltage used for constant voltage anodizing treatment is 20 V to 150 V; more preferably, the voltage used for constant voltage anodizing treatment is 20 V to 120 V; further preferably, the voltage used for constant voltage anodizing treatment is 20 V to 80 V; and furthermore preferably, the voltage used for constant voltage anodizing treatment is 30 V to 50 V

Preferably, in the preparation method of the alumina membrane, in the step (1), the constant voltage anodizing treatment is carried out for 24-72 hours; more preferably, the constant voltage anodizing treatment is carried out for 36-60 hours; still more preferably, the constant voltage anodizing treatment is carried out for 46-50 hours; and furthermore preferably, the constant voltage anodizing treatment is carried out for 47-49 hours.

Preferably, in the preparation method of the alumina membrane, in the step (1), the electrolyte used in the constant voltage anodizing treatment comprises at least one of sulfuric acid, oxalic acid, phosphoric acid, chromic acid, malonic acid, citric acid and malic acid. The electrolyte according to the present disclosure comprises at least one acid solution of sulfuric acid, oxalic acid, phosphoric acid, chromic acid, malonic acid, citric acid and malic acid and a mixed acid solution thereof, or a mixed solution of PEG-400 and one or more of sulfuric acid, oxalic acid, phosphoric acid, chromic acid, malonic acid, citric acid and malic acid. In some preferred embodiments of the present disclosure, the electrolyte is an oxalic acid solution. The alumina membrane prepared by using the oxalic acid as the electrolyte further reduces the adsorption of the alumina membrane on platelets, and the effect of transporting inflammatory factors is the best after attaching to the renal tubular cells, which may be related to a cell microenvironment.

More preferably, a concentration of the electrolyte is 0.1-1 mol/L; and furthermore preferably, a concentration of the electrolyte is 0.3-0.6 mol/L.

Preferably, in the preparation method of the alumina membrane, in the step (2), the physical processing method is conducted by using one of a fiber laser marking machine, an ultraviolet laser marking machine, a laser cutting machine, a numerical control engraving machine and an electric mill etching machine.

Preferably, in the preparation method of the alumina membrane, in the step (2), the pore-enlarging treatment comprises immersing the alumina membrane in a pore-enlarging solution; and more preferably, the pore-enlarging solution comprises one of a phosphoric acid solution and a sodium hydroxide solution.

More preferably, the pore-enlarging solution is a phosphoric acid solution; still more preferably, a mass fraction of the phosphoric acid solution is $\geq$ 1%; furthermore preferably, a mass fraction of the phosphoric acid solution is 1%-50%; still furthermore preferably, a mass fraction of the phosphoric acid solution is 2%-10%; in some preferred embodiments of the present disclosure, a mass fraction of phosphoric acid solution is 4%-6%.

More preferably, the alumina membrane is immersed in the pore-enlarging solution at a temperature of $\geq$ 4°C; still more preferably, the alumina membrane is immersed in the pore-enlarging solution at a temperature of 4-80°C; and furthermore preferably, the alumina membrane is immersed in the pore-enlarging solution at a temperature of 20-60°C.

More preferably, the alumina membrane is immersed in the pore-enlarging solution for $\geq$ 15 minutes; still more preferably, the alumina membrane is immersed in the pore-enlarging solution for 15-150 minutes; furthermore preferably, the alumina membrane is immersed in the pore-enlarging solution for 30-90 minutes; and still furthermore preferably, the alumina membrane is immersed in the pore-enlarging solution for 40-50 minutes.

Preferably, in the preparation method of the alumina membrane, in the step (3), after the pore-enlarging treatment, the alumina membrane is washed with ultrapure water, and then blew with $N_2$ for drying to obtain a porous alumina membrane with double channels.

A second aspect of the present disclosure provides an alumina membrane, and the alumina membrane is prepared by the preparation method of the alumina membrane as described above.

Preferably, a thickness of the alumina membrane is 10-100 $\mu$m; more preferably, a thickness of the alumina membrane is 15-75 $\mu$m; and still more preferably, a thickness of the alumina membrane is 30-60 $\mu$m.

Preferably, the alumina membrane has a porous structure with a pore diameter of 15-240 nm; more preferably, a pore diameter of 15-150 nm; still more preferably, a pore diameter of 50-150 nm; and furthermore preferably, a pore diameter of 60-120 nm.

Preferably, a thickness of the silicon coating on the alumina membrane is 5-100 nm; more preferably, a thickness of the silicon coating is 10-50 nm; and still more preferably, a thickness of the silicon coating is 15-30 nm.

Preferably, in the alumina membrane, the silicon coating comprises at least one of silicon dioxide, silicon nitride and polysilicon; and more preferably, the silicon coating is silicon dioxide.

A third aspect of the present disclosure provides an alumina biofilm comprising the alumina membrane as described above, wherein a surface on one side of the alumina membrane without depositing the silicon coating is attached with renal tubule epithelial cells.

Preferably, a loading amount of the renal tubule epithelial cells on the alumina biofilm is $5*10^2$-$5*10^5$ cells/cm$^2$; more preferably, a loading amount of the renal tubule epithelial cells is $5*10^3$-$5*10^5$ cells/cm$^2$; and still more preferably, a loading amount of the renal tubule epithelial cells is $5*10^4$-$5*10^5$ cells/cm$^2$.

A fourth aspect of the present disclosure provides use of the alumina membrane and the alumina biofilm as described above in preparing a hemofiltration device, a hemodialysis device, a sepsis prevention and treatment device and an inflammatory factor transporting device.

The present disclosure has the beneficial effects as follows.

In the preparation method of the alumina membrane according to the present disclosure, pure aluminum on the surface on the other side is etched by the physical processing method after the surface on one side is oxidized, instead of the chemical etching method, which avoids an absorbability of an alumina crystal form formed by chemical reagent corrosion on a platelet membrane protein; by using the deposited silicon coating, the present disclosure can effectively reduce the adsorption of the platelets, and meanwhile, promote an inflammatory factor absorbing and transporting capacity and a reabsorption capacity of the cells on the surface on other side (the side in which dialysate is located).

The alumina membrane according to the present disclosure directly provides a suitable microenvironment for the growth of the renal tubule epithelial cells, and does not need to additionally use biological materials (such as porous microbeads) to prepare a renal tubule assist device, so as to maintain an efficient protein (inflammatory factor) transporting capacity of the renal tubule epithelial cells.

The alumina membrane according to the present disclosure has small absorbability on the platelets, and is not hemolytic. Moreover, it is not easy to be blocked when the alumina membrane is used as a semi-permeable membrane/dialysis membrane for hemodialysis or hemofiltration.

The alumina membrane according to the present disclosure has high hydrophilicity, high biocompatibility and a high cell adhesion rate.

## BRIEF DESCRIPTION OF DRAWINGS

FIG. 1 is a scanning electron microscope image of a back surface (reverse surface) of an alumina membrane in Example 1 before depositing silicon dioxide.

FIG. 2 is a scanning electron microscope image of a back surface (reverse surface) of an alumina membrane in Example 1 after depositing silicon dioxide.

FIG. 3 is a scanning electron microscope image of a back surface (reverse surface) of an alumina membrane in Comparative Example 1 before depositing silicon dioxide.

FIG. 4 is a scanning electron microscope image of a back surface (reverse surface) of an alumina membrane in Comparative Example 1 after depositing silicon dioxide.

FIG. 5 is a scanning electron microscope image of a back surface (reverse surface) of an alumina membrane in Comparative Example 3 before depositing silicon dioxide.

FIG. 6 is a scanning electron microscope image of a back surface (reverse surface) of an alumina membrane in Comparative Example 3 after depositing silicon dioxide.

FIG. 7 is a schematic diagram of an alumina -fixed culture box device in Example 2.

FIG. 8 is a viability staining diagram of cells inoculated on the alumina membrane in Example 1 as described in Example 3.

FIG. 9 is a viability staining diagram of cells inoculated on the alumina membrane in Comparative Example 1 as described in Example 3.

FIG. 10 is a viability staining diagram of cells inoculated on the alumina membrane in Comparative Example 2 as described in Example 3.

FIG. 11 is a viability staining diagram of cells inoculated on the alumina membrane in Comparative Example 3 as described in Example 3.

FIG. 12 is a viability staining diagram of cells inoculated on the alumina membrane in Comparative Example 4 as described in Example 3.

FIG. 13 is a schematic diagram of a device for removing sepsis toxin by using an alumina membrane in Example 4.

## DETAILED DESCRIPTION

The concept and technical effects of the present disclosure will be described clearly and completely hereinafter with reference to the examples, so as to fully understand the objectives, features and effects of the present disclosure. Obviously, the described example is only a part of the examples of the present disclosure, but not all the examples. Based on the examples of the present disclosure, other examples obtained by those skilled in the art without creative labor shall fall into the protection scope of the present disclosure.

Example 1

A method for preparing an alumina membrane of this example comprised the following steps of

(1) carrying out degreasing treatment on an aluminum sheet with a thickness of 0.2 mm and a length and width of 10 cm× 10 cm, immersing the aluminum sheet in ethanol for 5 minutes, and then placing the aluminum sheet in an electrolytic cell for electrochemical polishing with a constant current method to obtain an aluminum sheet with a bright and flat surface; wherein the electrolyte was an ethanol solution of 100 g/L of phosphoric acid and 40 g/L of polyethylene glycol-400, a current value was set at 2.63 A, a voltage was not more than 130 V, and the polishing lasted for about 15 minutes.

(2) Using an aluminum sheet and a platinum sheet with the same area respectively as an anode and a cathode to form two electrodes; covering the anode aluminum sheet with a fixture to expose one side surface (called a front surface), wherein front surface was in contact with the electrolyte, and the surface on other side covered by the fixture (called a back surface or a reverse surface) was not in contact with the electrolyte; immersing the two electrodes in a 0.3 M of oxalic acid electrolyte, and carrying out anodizing treatment at 40 V for 48 hours, thus obtaining an alumina membrane with a porous structure.

(3) Taking out the alumina membrane with the porous structure, etching the reverse surface of the alumina membrane with the porous structure by using a fiber laser marking machine to etch off the pure aluminum, to obtain a large piece of intact alumina membrane without any damage.

(4) Immersing the alumina membrane prepared in the step (3) in a phosphoric acid solution with a mass fraction of 5% at 45°C for about 45 minutes, then washing the alumina membrane with a large amount of ultrapure water, and drying the alumina membrane with $N_2$ to obtain the alumina membrane with interconnected pores on the front surface and back surface.

(5) Depositing silicon dioxide on the reverse surface of the alumina membrane by a plasma enhanced chemical vapor deposition method technology to obtain a silicon dioxide coating with a thickness of 20 nm by controlling a reaction time finally, i.e., the alumina membrane b of this example, wherein a substrate had a temperature of 300°C, a radio-frequency power was 200 W, an flow rate of $N_2$ was 200 sccm, an flow rate of $SiH_4$ was 11 sccm, an flow rate $N_2O$ of was 37 sccm, and a gas pressure was 1,000 mT.

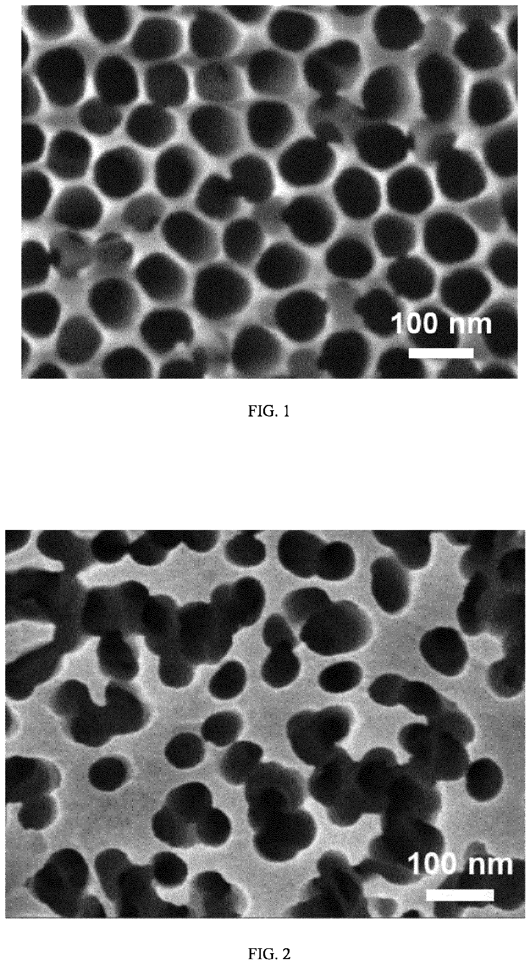

In the step (5) of this example, a scanning electron microscope image of the back surface (reverse surface) of the porous alumina membrane before depositing silicon dioxide was shown in FIG. 1; and a scanning electron microscope image after depositing silicon dioxide was shown in FIG. 2.

In the preparation method of the alumina membrane, an area of an alumina membrane and a number of aluminum sheets used for oxidation in one technology could be controlled by adjusting a size and a number of openings exposed to the electrolyte in the fixture. In addition, circular, square or runner-shaped alumina membrane could be prepared by controlling a laser etching technology.

Comparative Example 1

The differences between the preparation method of the alumina membrane in this comparative example and that in Example 1 were that: the step (3) was different, and the other steps were the same as those in Example 1. The step (3) of this comparative example was: chemically etching the alumina membrane with the porous structure obtained in the step (2), and using a mixed solution of 0.3 M of $CuCl_2$ and 10wt% of HCl to etch off the pure aluminum at a room temperature to obtain the alumina membrane with through holes.

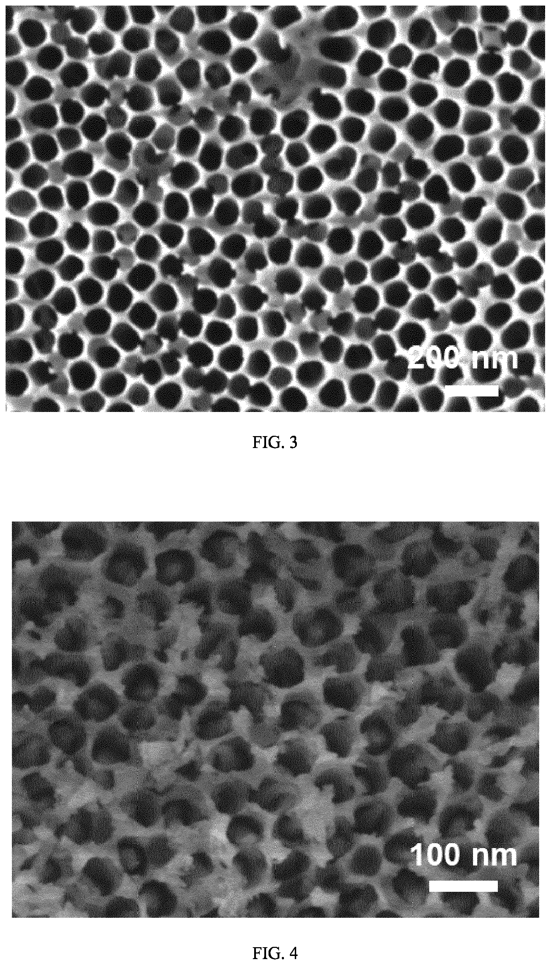

In the step (5) of this comparative example, a scanning electron microscope image of the back surface (reverse surface) of the alumina membrane before depositing silicon dioxide was shown in FIG. 3; and a scanning electron microscope image after depositing silicon dioxide was shown in FIG. 4.

Comparative Example 2

The differences between the preparation method of the alumina membrane in this comparative example and that of Example 1 were that: the step (5) of Example 1 was not conducted in Comparative Example 2, that was, an alumina membrane without depositing silicon dioxide was obtained.

Comparative Example 3

The differences between the preparation method of the alumina membrane in this comparative example and that in Example 1 were that: the step (2) was different, and the other steps were the same as those in Example 1. In the step (2) of this comparative example, 6 M of phosphoric acid solution was used as an electrolyte, and the anodic oxidation was carried out at a voltage of 100 V to obtain an alumina membrane.

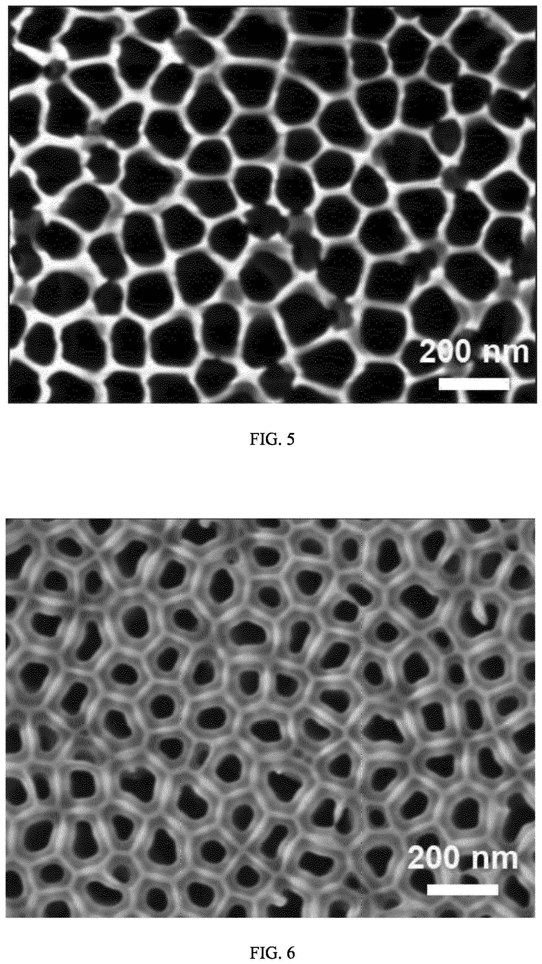

In the step (5) of this comparative example, a scanning electron microscope image of the back surface (reverse surface) of the alumina membrane before depositing silicon dioxide was shown in FIG. 5; and a scanning electron microscope image after depositing silicon dioxide was shown in FIG. 6.

Comparative Example 4

The differences between the preparation method of the alumina membrane in this comparative example and that in Example 1 were that: the step (5) was different, and the other steps were the same as those in Example 1. In the step (5) of this comparative example, silicon dioxide was deposited on the front surface of the alumina membrane by a plasma enhanced chemical vapor deposition method.

Comparative Example 5

The differences between the preparation method of the alumina membrane in this comparative example and that in Example 1 were that: the step (3) in Example 1 was not conducted in Comparative Example 5, and the other steps were the same as those in Example 1. In the step (2) of this comparative example, the reverse surface of the porous alumina membrane was not etched by a fiber laser marking machine, but the step (4) of pore-enlarging by using phosphoric acid was directly carried out.

A result showed that because an aluminum elementary substance layer was in the middle of the membrane, the pore-enlarging could not be completed by using phosphoric acid, and the pores on the front surface and the back surface of the membrane were not communicated.

Pore parameters of the alumina membranes prepared in the step (5) in Example 1 and Comparative Examples 1-4 were shown in Table 1 below.

Table 1

|  | Pore diameter size after depositing silicon dioxide (nm) | Thickness of membrane ($\mu$m) | Thickness of silicon dioxide coating (nm) |
|---|---|---|---|
| Example 1 | 80-100 | $50\pm5$ | 20 |
| Comparative Example 1 | 80-100 | $50\pm5$ | 20 |
| Comparative Example 2 | 80-100 | $50\pm5$ | 0 |
| Comparative Example 3 | 80-100 | $25\pm3$ | 20 |
| Comparative Example 4 | 80-100 | $50\pm5$ | 20 |

Example 2

Blood compatibility and water contact angle test of alumina membrane

When the alumina membrane is used in blood filtration, hemodialysis and other aspects, the reverse surface (back surface) of the alumina membrane is in contact with blood, while the front surface of the alumina membrane is in contact with dialysate, so certain requirements are put forward on the blood compatibility of the reverse surface (silicon coating) and the hydrophilicity of the front surface (for attaching renal tubular cells) of the alumina membrane.

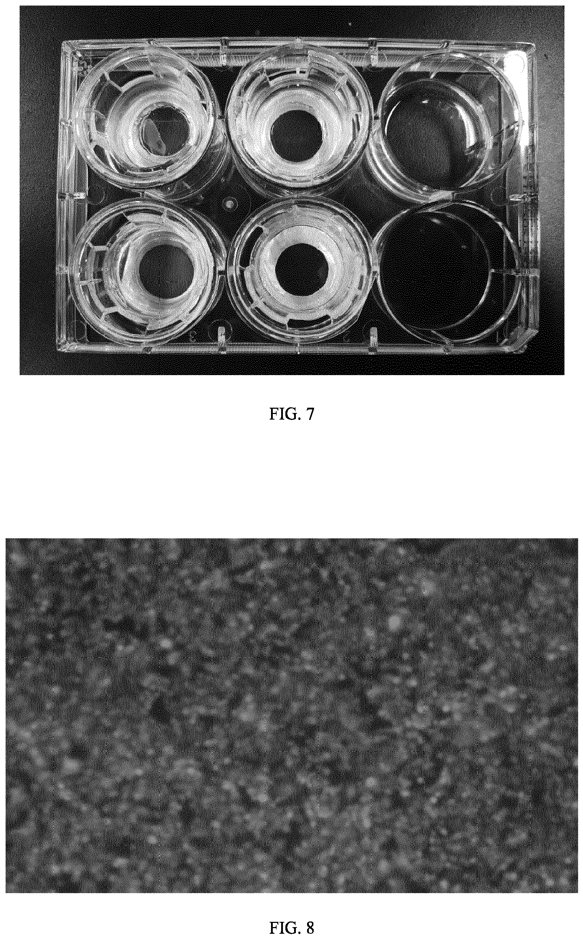

Blood compatibility test: the alumina membranes prepared in the step (5) in Example 1 and Comparative Examples 1-4 were fixed in a culture box respectively, as shown in FIG. 7, with the reverse surface of the alumina membranes facing upward (in short, for Example 1, and Comparative Example 1 and Comparative Example 3, the reverse surface were the surfaces with the silicon dioxide coating; for Comparative Example 2 and Comparative Example 4, the reverse surface were the surfaces etched by the fiber laser marking machine without silicon coating). 1mL of plasma (containing about $20*10^6$ rabbit platelets) was added to the reverse surface of the alumina membrane respectively, and the culture box was sealed with paraffin. After the culture box was oscillated at 37°C for 1 hour, the membrane was moistened with a physiological saline solution for three times, blood components on the membrane were fixed with 3% of glutaraldehyde aqueous solution, washed with ultrapure water, and then dried. The samples were observed by a scanning electron microscope, and a number of the platelets attached to the membrane in a visual field (about $1 \times 10^3$ $\mu m^2$) were counted. An average number of the platelets attached to the membrane in ten different visual fields was taken as a number of platelets attached.

Hydrophilicity test: according to a sessile drop method in GB/T 30447-2013 *Measurement Method for Contact Angle of Nano-film Surface,* a contact angle between water droplets and the front surface of the alumina membrane was calculated by using a shape of a water droplet for detection.

Each group was measured three times, and final measurement results were calculated according to an average value of the three measurement results. The hydrophilicity (contact angle) and the absorption number of rabbit platelets were shown in Table 2 below.

Table 2

| | Example 1 | Comparative Example 1 | Comparative Example 2 | Comparative Example 3 | Comparative Example 4 |
|---|---|---|---|---|---|
| Contact angle of front surface of membrane (°) | 42±3 | 38±4 | 46±3 | 43±3 | 26±2 |
| Absorption number of rabbit platelets on reverse surface of membrane ($/10^3$ cm$^2$) | 8±0.43 | 43±2.9 | 17±1.1 | 25±2.6 | 15±0.7 |

As shown in Table 2, the contact angles of the alumina membranes of Example 1 and Comparative Example 1 to Comparative Example 3 were not much different, and all could be wetted. It was reported that a surface of silicon dioxide was rich in hydroxyl groups, which could combine with molecules in a form of hydrogen bonds to form a multi-molecular monolayer adsorption layer, thus producing certain chemical adsorption on the platelets. However, comparing with Example 1, Comparative Example 2 and Comparative Example 4, it could be seen that the alumina membrane with the silicon dioxide coating on the reverse surface as described in Example 1 absorbed less platelets, which may be because silicon dioxide was negatively charged in a blood environment, and the platelets were negatively charged, so that the silicon dioxide and the platelets were mutually exclusive. In Comparative Example 1, the alumina membrane prepared by chemical etching through holes had the largest adsorption amount of platelet, which might be due to the stronger adsorption of the platelets by the crystal form and surface chemical properties of the alumina formed by chemical etching. Compared with Comparative Example 3 and Example 1, due to different electrolytes, the crystal forms and/or microstructures of membrane are different, which in turn leaded to different adsorption numbers of rabbit platelets.

Example 3

Cell activity test

The alumina membranes in Example 1 and Comparative Examples 1-4 were washed with a PBS solution for 1-2 times, and renal tubule epithelial cells were inoculated on the surfaces of the alumina membranes, respectively. Human renal tubule epithelial cells were inoculated on the front surface of each membrane with a density of $1*10^5$ cells/cm$^2$ (the membrane area was 2 cm$^2$), and the cells were left in an incubator for 4 hours until the cells attached to the wall. The cells on the membrane were detected as follows:

1. Using a sterile PBS to wash the membrane for 1-2 times, adding a detection liquid (comprising a culture medium and a CCK-8 reagent which were evenly mixed in a volume ratio of 10: 1), then incubating in an incubator for 1-2 hours, collecting the detection liquid in a 96-well plate, and detecting an absorbance value of the detection liquid with an microplate reader. In addition, a blank control group, a negative control group and a positive control group were set.

2. Staining the cells on the membrane with a Calcein-AM/PI double staining solution, and observing the viability and morphology of cells by a fluorescence microscope.

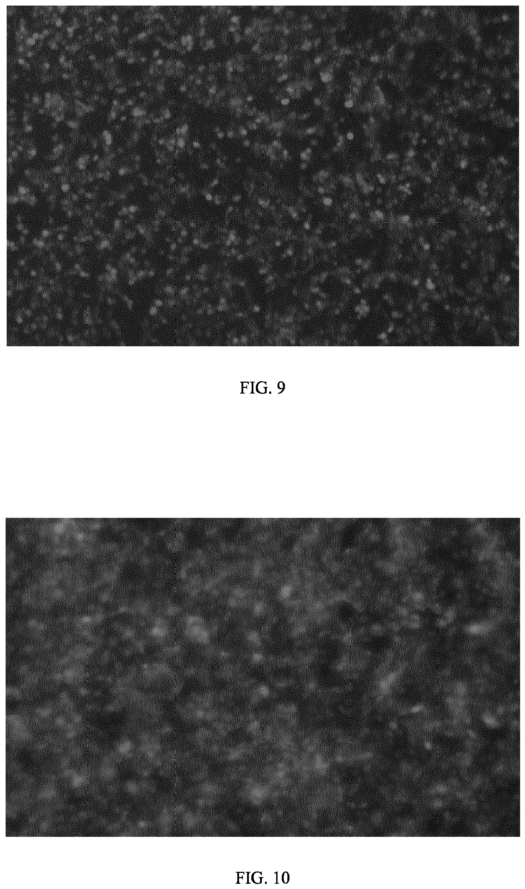

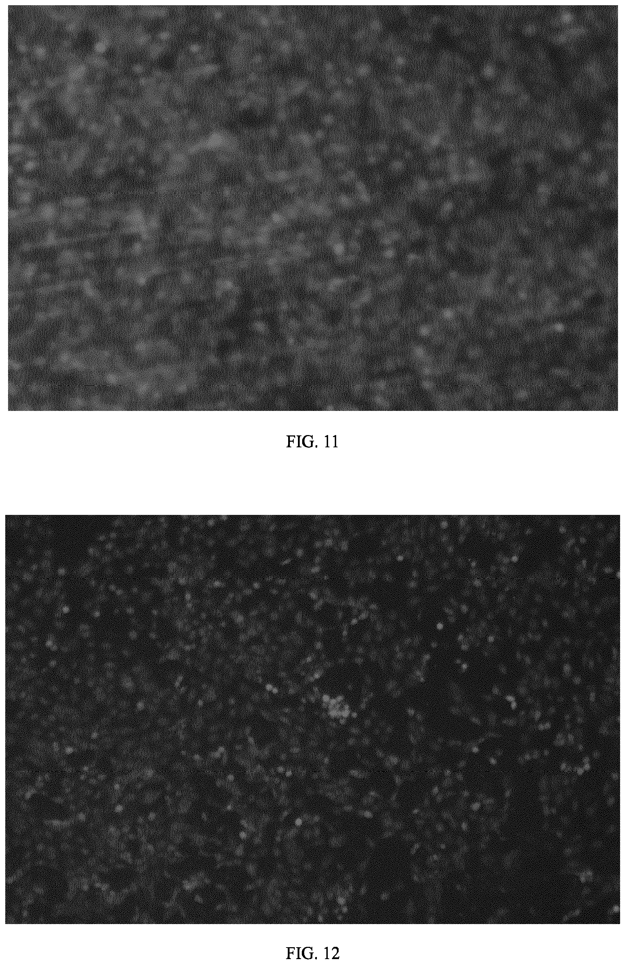

3. Washing the membrane with the PBS for 2-3 times, labeling the cells on the membrane with a specific antibody labeling (FITC), digesting the cells, collecting, and analyzing by a flow analysis to obtain a cell adhesion rate according to a ratio of an amount of the cells attached to the membrane to a total amount of the cells grown. Each group was measured three times, and an average adhesion rate was calculated according to an average value of the three measurement results. The results were shown in Table 3. The viability staining diagrams of cells were shown in FIG. 8 to FIG. 12 respectively after the alumina membranes prepared in Example 1 and Comparative Example 1 to Comparative Example 4 were inoculated with cells.

Table 3

| Group | Example 1 | Comparative Example 1 | Comparative Example 2 | Comparative Example 3 | Comparative Example 4 |
|---|---|---|---|---|---|
| Average adhesion rate of renal tubule epithelial cells (%) | 82±2.5 | 73±5.6 | 81±3.2 | 83±1.9 | 65±3.5 |

The cell adhesion rate in Comparative Example 1 was relatively low, which might be due to a situation that the pure aluminum was not removed by a physical method, but by chemical etching. The cell adhesion rate in Comparative Example 4 was relatively low because the biocompatibility of silicon dioxide to the cells was not as good as that of alumina. The cell adhesion rate of the alumina membrane in Comparative Example 4 was relatively low, so a follow-up test would not be continued.

Example 4

Removal of sepsis toxin by alumina membrane

① Preparation of sepsis plasma:

(1) taking 92.5 mmol of NaCl, 2.5 mmol of $CaCl_2$, 1.1 mmol of $MgCl_2$, 4.3 mmol of KCl, 24 mmol of $NaHCO_3$, 2.5 mmol of $NaH_2PO_4$ and 11.5 mmol of $Na_2HPO_4$, and adding water to prepare 1L of inorganic salt solution.
(2) adding glucose and amino acid into the inorganic salt solution, with working concentrations of 990 mg/L and 600 mg/L respectively.
(3) adding creatinine (a final concentration of 120 $\mu$mol/L) and urea (a final concentration of 5 mmol/L) to dissolve, then adding a septic blood marker IL-6 (a final concentration of 300 ng/L), and finally adding plasma albumin (a final concentration of 60 g/L) and $\beta$2-microglobulin (a final concentration of 2 mg/L) to adjust a colloid osmotic pressure of a resulting solution, so as to obtain a simulated sepsis plasma of a sepsis patient.

② Preparation of alumina biofilms (cell culture):
Inoculating renal tubule epithelial cells at a density of $1*10^5$ cells/cm$^2$ on the front surfaces of the alumina membranes (the membrane had an area of 100 cm$^2$) in Example 1 and Comparative Examples 1-3 respectively, and culturing the cells for 24 hours to form alumina biofilms.
③ Inflammatory factor clearance test:

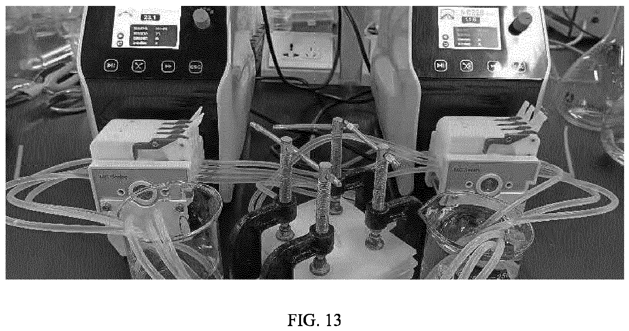

Clamping the described alumina biofilms loaded with the renal tubule epithelial cells respectively in a special fixture device (equivalent to a dialysis device), establishing two diaphragm chambers by separating with the alumina membranes, and forming a circulation system with the two diaphragm chambers, a peristaltic pump and a pipeline respectively. The device was shown in FIG. 13.
The reverse surface of the membrane was in contact with the simulated sepsis plasma, while the front surface of the membrane (the surface attaching to renal tubule epithelial cells) was in contact with the commercially available blood dialysate, in which exchange of substances between the two sides of the membrane could only be carried out through the membrane as a medium. Flow velocity on both sides of the membrane was set as below: a flow velocity of the simulated sepsis plasma of 20 mL/min, a flow velocity of the blood dialysate of 10 mL/min, and a circulation time of 24 hours. At 24 hours, samples were taken to detect a content of IL-6 (human interleukin-6) in a dialysate and a volume of the dialysate. An IL-6 ELISA detection kit was used for quantitatively detecting the IL-6 on the side of dialysate, and a light absorption value A at $\lambda$=450 nm was collected by a microplate reader instrument. An average light absorption value of a standard substance, a blank control and a sample was read, and the average light absorption value of the blank control was subtracted to obtain a light absorption calibration value of the standard substance and the sample. A standard curve was drawn by taking a concentration of the standard substance as a horizontal axis and a light absorption value of the calibrated standard substance as a vertical axis. A clearance rate of the IL-6 was calculated according to a ratio of an increasing amount of the IL-6 on the side of dialysate to a total amount of the IL-6 contained in the original plasma, and each group was measured three times. An average clearance rate of the IL-6 was calculated according to an average value of the three measurement results.
The clearance rate of the IL-6 was calculated according to the following formula:

$$Clearance\ rate\ \%\ of\ \text{IL–}6 = \frac{C_t \times V_t}{C_0 \times V_0} \times 100\%$$

Ct was a concentration corresponding to an absorbance of the dialysate at $\lambda$=450 nm at a time of t, and $V_t$ was a volume of the dialysate after circulation for a time of t =24 hours;

$C_0$ was a concentration corresponding to an absorbance of the original plasma at $\lambda$=450 nm at a time of 0, and $V_0$ was a total volume of the original plasma.

The clearance rate of the IL-6 was shown in Table 4.

Table 4

| Group | Example 1 | Comparative Example 1 | Comparative Example 2 | Comparative Example 3 |
|---|---|---|---|---|
| Average clearance rate of IL-6 (%) | 35.1±1.4 | 26.9±0.7 | 28.7±0.5 | 29.1±1.2 |

According to the results in Table 3, the cell adhesion rates of the alumina membranes in Example 1, Comparative Example 2 and Comparative Example 3 were not much different in a culture medium environment, which might be because the culture medium environment was relatively stable and the cell state showed little difference. However, in an environment where a dialysate flowed, and the dialysate contained the inflammatory factors and other factors, the state performance of the cells on different alumina membranes was quite different. As shown in Table 4, the clearance rate of the IL-6 by the renal tubule epithelial cells on the alumina membrane in Example 1 was significantly higher than that of alumina membranes in other Comparative Examples, which indirectly reflected that in a hemodialysis/hemofiltration environment, the renal tubular cells on the alumina membrane in Example 1 still maintained a relatively good state and an ability to transport proteins was still relatively strong. This might be because the microenvironment provided by the alumina biofilm in Example 1 was more suitable for the renal tubule epithelial cells. The results showed that the coating, the electrolyte and the aluminum removing technology would all affect the microenvironment of the alumina membrane and the uptake and transport of the cells.

Example 5

Reabsorption of $\beta$2-MG protein by alumina biofilm

$\beta$2-microglobulin ($\beta$2-MG) protein is a small molecule globulin produced by lymphocytes, platelets and polymorphonuclear leukocytes, with a molecular weight of 11,800. In normal people, $\beta$2-MG is freely filtered from glomerulus, and 99.9% of $\beta$2-MG is absorbed in a proximal renal tubule, so that renal tubule epithelial cells also plays an important role in the reabsorption of $\beta$2-MG, which is of great therapeutic significance to a sepsis patient.

A reabsorption capacity of the biofilm on protein depends on a total amount of microglobulin on the side of dialysate that is reabsorbed back to the plasma by passing through the cells on the biofilm. In a reabsorption experiment, the preparation of sepsis plasma, cell culture, circulating perfusion device (experimental device) and the flow velocity on both sides of the membrane were set as those in Example 4, while creatinine (a final concentration of 120 $\mu$mol/L) and urea (a final concentration of 5 mmol/L) were added to the dialysate, then a septic blood marker IL-6 (a final concentration of 300 ng/L) was added, finally plasma albumin (a final concentration of 60 g/L) and $\beta$2-microglobulin ( final concentration of 2 mg/L) were added, and the dialysate with the same concentration of the $\beta$2-MG protein as the sepsis plasma was obtained while adjusting a solution colloid osmotic pressure.

A content of $\beta$2-MG on the side of dialysate was detected by sampling at a circulation time point of 24 hours, and the $\beta$2-MG ELISA detection kit was used for quantitatively detecting $\beta$2-MG on the side of dialysate, and the light absorption value A at $\lambda$=450 nm was collected by a microplate reader instrument. A total reabsorption amount of $\beta$2-MG by the cells was calculated according to the light absorption value and the volume of dialysate. An average light absorption value of a standard substance, a blank control and a sample was taken, and the average light absorption value of the blank control was subtracted to obtain a light absorption calibration value of the standard substance and the sample. A standard curve was drawn by taking a concentration of the standard substance as a horizontal axis and a light absorption value of the calibrated standard substance as a vertical axis. Each group was measured three times, and finally the average reabsorption amount of the microglobulin by the membrane was calculated according to the average value of the three measurement results.

The total reabsorption amount of β2-MG protein was calculated according to a total amount of β2-MG protein reduced on the side of dialysate, and the formula was as follow:

$$\text{Reabsorption amount of } \beta2 - \text{MG protein} = \frac{C_0 \times V_0 - C_t \times V_t}{kb}$$

$C_0$ was a concentration corresponding to the absorbance of dialysate at $\lambda$=450 nm at a time of 0, and $V_0$ was a volume of dialysate at a time of 0;

Ct was a concentration corresponding to the absorbance of dialysate at $\lambda$=450 nm at a time of t, and $V_t$ was a volume of dialysate at a time of t;

k was a molar absorption coefficient, and b was a thickness of the absorption layer, i.e.,1 cm.

The total reabsorption amount of β2-MG protein by the bio-film was shown in Table 5.

Table 5

| Group | Example 1 | Comparative Example 1 | Comparative Example 2 | Comparative Example 3 |
|---|---|---|---|---|
| Average reabsorption amount of β2-MG (mg) | 1.48±0.17 | 0.58±0.07 | 0.97±0.11 | 1.25±0.13 |

According to data in Table 5 above, the alumina could effectively promote the reabsorption of the β2-MG protein by the cells. Particularly, the alumina membrane prepared by the method in Example 1 could effectively promote a reabsorption function of the β2-MG protein by the cells on the membrane. In addition, silicon dioxide was deposited on the blood side of the membrane, which effectively reduced the adhesion of the membrane to protein and thus improved a material exchange efficiency and a total filtration amount of the membrane to protein.

Those described above are only the preferred examples of the present disclosure, but are not used for limiting the present disclosure. For those skilled in the art, the present disclosure may have various modifications and changes. Any modification, equivalent substitution and improvement made within the spirit and principle of the present disclosure shall fall within the protection scope of the present disclosure.

**Claims**

1. A method for preparing an alumina membrane comprises the following steps of

(1) carrying out constant voltage anodizing treatment on a surface on one side of an aluminum sheet to obtain an alumina membrane with a porous structure on the surface on one side;
(2) removing pure aluminum on an other side of the alumina membrane prepared in the step (1) by a physical processing method, and carrying out pore-enlarging treatment to obtain a membrane with interconnected pores on both sides; and
(3) depositing a silicon coating on the surface of the membrane on the side that has been physically processed in the step (2) to obtain the alumina membrane.

2. The method for preparing the alumina membrane according to claim 1, wherein in the step (1), a voltage used for the constant voltage anodizing treatment is 20-150 V

3. The method for preparing the alumina membrane according to claim 1, wherein in the step (1), an electrolyte used in the constant voltage anodizing treatment comprises at least one of sulfuric acid, oxalic acid, phosphoric acid, chromic acid, malonic acid, citric acid and malic acid.

4. The method for preparing the alumina membrane according to claim 3, wherein a concentration of the electrolyte is 0.1-1 mol/L.

5. An alumina membrane, prepared by the method for preparing the alumina membrane according to any one of claims 1 to 4.

6. The alumina membrane according to claim 5, wherein a thickness of the alumina membrane is 10-100 $\mu$m.

7. The alumina membrane according to claim 5, wherein the silicon coating of the alumina membrane comprises at least one of silicon dioxide, silicon nitride and polysilicon.

8. An alumina biofilm, comprising the alumina membrane according to any one of claims 5 to 7, wherein a surface on one side of the alumina membrane without depositing the silicon coating is attached with renal tubule epithelial cells.

9. The alumina biofilm according to claim 8, wherein a loading amount of the renal tubule epithelial cells on the alumina biofilm is $5*10^2$-$5*10^5$ cells/cm$^2$.

10. Use of the alumina membrane according to any one of claims 5 to 7 and/or the alumina biofilm according to any one of claims 8 to 9 in preparing a hemofiltration device, a hemodialysis device, a sepsis prevention and treatment device and an inflammatory factor transporting device.

FIG. 1

FIG. 2

FIG. 3

FIG. 4

FIG. 5

FIG. 6

FIG. 7

FIG. 8

FIG. 9

FIG. 10

FIG. 11

FIG. 12

FIG. 13

## INTERNATIONAL SEARCH REPORT

| International application No. |
|---|
| **PCT/CN2023/077426** |

### A. CLASSIFICATION OF SUBJECT MATTER

C23C28/04(2006.01)i

According to International Patent Classification (IPC) or to both national classification and IPC

### B. FIELDS SEARCHED

Minimum documentation searched (classification system followed by classification symbols)

IPC:C23C,C25D

Documentation searched other than minimum documentation to the extent that such documents are included in the fields searched

Electronic data base consulted during the international search (name of data base and, where practicable, search terms used)

CNABS, CNKI, CNTXT: 多孔, 氧化铝, 硅, 阳极氧化, 剥离, 物理, 机械, 激光打标, 激光切割, 数控雕刻, 电磨机蚀刻; DWPI, VEN, ELSEVIER: porous, alumina, silicon, silica, anodic oxidation, peel, physical processing, machining, mechanical, laser marking, laser cutting, cnc engraving, electric mill etching

### C. DOCUMENTS CONSIDERED TO BE RELEVANT

| Category* | Citation of document, with indication, where appropriate, of the relevant passages | Relevant to claim No. |
|---|---|---|
| PX | CN 115369404 A (SHENZHEN HUAYUE REGENERATIVE MEDICINE BIOTECHNOLOGY CO., LTD. et al.) 22 November 2022 (2022-11-22)<br>claims 1-10 | 1-10 |
| X | CN 1669920 A (ZHEJIANG UNIVERSITY) 21 September 2005 (2005-09-21)<br>claims 1-2, and embodiment 2 | 5-7, 10 |
| Y | CN 1669920 A (ZHEJIANG UNIVERSITY) 21 September 2005 (2005-09-21)<br>claims 1-2, and embodiment 2 | 1-4, 8-9 |
| Y | CN 101597639 A (INSTITUTE OF SEMICONDUCTORS, CHINESE ACADEMY OF SCIENCES) 09 December 2009 (2009-12-09)<br>description, page 2 | 1-4 |
| Y | US 2020392639 A1 (NANOPEC INC.) 17 December 2020 (2020-12-17)<br>description, abstract and description, paragraph 5 | 8-9 |
| A | CN 101441192 A (HEFEI INSTITUTES OF PHYSICAL SCIENCE, CHINESE ACADEMY OF SCIENCES) 27 May 2009 (2009-05-27)<br>entire document | 1-10 |

☑ Further documents are listed in the continuation of Box C.    ☑ See patent family annex.

| | |
|---|---|
| *   Special categories of cited documents:<br>"A"   document defining the general state of the art which is not considered to be of particular relevance<br>"D"   document cited by the applicant in the international application<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| **17 May 2023** | **19 May 2023** |

| Name and mailing address of the ISA/CN | Authorized officer |
|---|---|
| **China National Intellectual Property Administration (ISA/ CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
|---|
| **PCT/CN2023/077426** |

**C. DOCUMENTS CONSIDERED TO BE RELEVANT**

| Category* | Citation of document, with indication, where appropriate, of the relevant passages | Relevant to claim No. |
|---|---|---|
| A | CN 102183629 A (INSTITUTE OF SEMICONDUCTORS, CHINESE ACADEMY OF SCIENCES) 14 September 2011 (2011-09-14)<br>entire document | 1-10 |
| A | CN 104233430 A (XI'AN INSTITUTE OF OPTICS AND PRECISION MECHANICS OF THE CHINESE ACADEMY OF SCIENCES) 24 December 2014 (2014-12-24)<br>entire document | 1-10 |
| A | CN 111212922 A (POLYTECHNIC UNIVERSITY OF VALENCIA et al.) 29 May 2020 (2020-05-29)<br>entire document | 1-10 |

Form PCT/ISA/210 (second sheet) (July 2022)

**INTERNATIONAL SEARCH REPORT**
Information on patent family members

International application No.

**PCT/CN2023/077426**

| Patent document cited in search report | | | Publication date (day/month/year) | Patent family member(s) | | | Publication date (day/month/year) |
|---|---|---|---|---|---|---|---|
| CN | 115369404 | A | 22 November 2022 | None | | | |
| CN | 1669920 | A | 21 September 2005 | None | | | |
| CN | 101597639 | A | 09 December 2009 | None | | | |
| US | 2020392639 | A1 | 17 December 2020 | WO | 2020257092 | A1 | 17 December 2020 |
| | | | | CN | 114007726 | A | 01 February 2022 |
| | | | | CN | 114007726 | B | 20 December 2022 |
| CN | 101441192 | A | 27 May 2009 | None | | | |
| CN | 102183629 | A | 14 September 2011 | None | | | |
| CN | 104233430 | A | 24 December 2014 | None | | | |
| CN | 111212922 | A | 29 May 2020 | CA | 3074815 | A1 | 14 March 2019 |
| | | | | SG | 11202001981 | YA | 29 April 2020 |
| | | | | US | 2021404020 | A1 | 30 December 2021 |
| | | | | EP | 3680345 | A1 | 15 July 2020 |
| | | | | EP | 3680345 | A4 | 06 October 2021 |
| | | | | JP | 2020537535 | A | 24 December 2020 |
| | | | | WO | 2019048722 | A1 | 14 March 2019 |
| | | | | KR | 20200104281 | A | 03 September 2020 |
| | | | | ES | 2702999 | A1 | 06 March 2019 |
| | | | | ES | 2702999 | B2 | 28 June 2022 |
| | | | | AU | 2018330613 | A1 | 19 March 2020 |

Form PCT/ISA/210 (patent family annex) (July 2022)

## REFERENCES CITED IN THE DESCRIPTION

**Patent documents cited in the description**

- US 6653131 B2